# EUROPEAN PATENT APPLICATION

(11) **EP 2 065 051 A2**
(43) Date of publication of application: **03.06.2009**
(21) Application number: 08015022.0
(22) Date of filing: 26.08.2008
(51) Int. Cl.: A61K 38/38, A61K 31/05, A61K 31/7024, A61P 1/12

(54) **Composition for maintaining gastrointestinal homeostasis comprising albumin and tannic acid**

(30) Priority: 29.08.2007 US 897156
(71) Applicant: KIEL LABORATORIES, INC., Gainesville, GA 30504 (US)
(72) Inventor: Kiel, Jeffrey S., Gainesville Georgia 30504 (US); Thomas, Gregory H., Carrollton Georgia 30117 (US); Todebush, Richard Andrew, Norcross Georgia 30092 (US)
(74) Representative: Ryan, Anne Mary

(57) **Abstract**

The invention relates to a suspension of albumin and tannic acid for maintaining gastrointestinal homeostasis.

## Description

### FIELD OF THE INVENTION

The present invention relates to a suspension of albumin and tannic acid for maintaining gastrointestinal homeostasis.

### BACKGROUND OF THE INVENTION

The gastrointestinal (GI) tract consists of a diverse set of organs that are often thought to simply function to receive, digest, absorb, and eliminate ingested substances. However, the GI tract is a highly sophisticated organ system that coordinates these functions, and the many other automated functions that it performs, with the largest collection of endocrine and immune cells in the body, as well as the second largest collection of neural cell bodies. Disorders of the GI tract are therefore diverse and can result from the altered homeostasis of any of these gastrointestinal tract components.

Diarrhea is a common cause of dehydration, both of which are known to alter GI homeostasis.

Diarrhea is a medical term used to describe a condition in which bowel movements increase in frequency, liquidity and volume. Diarrhea is usually described as loose, watery stools, and patients may also experience abdominal pain, abdominal cramping, fever, nausea or blood in the stool.

Diarrhea is extremely common in the elderly and in children. Diarrhea usually lasts a few days and does not typically require treatment. However, diarrhea can become life-threatening when it causes severe dehydration, especially in the elderly and children.

Different processes in the body may promote diarrhea. These include a failure of the intestines to absorb adequate nutrients and fluids during digestion, the release of too much fluid into the digestive tract or complications as the result of intestinal disorders.

Diarrhea can be short-term (acute) or long-term (chronic), and both types can range in severity. Acute diarrhea lasts less than four weeks. The most common cause of acute diarrhea is a viral infection (e.g., stomach flu) or bacteria or parasites consumed through contaminated food or water (e.g., food poisoning, traveler's diarrhea). Chronic diarrhea lasts for more than four weeks and may indicate a more serious problem, such as malabsorption of nutrients, inflammatory bowel disease (IBD) or irritable bowel syndrome (IBS).

There are many therapeutic agents available for the management of diarrhea. Several products currently popular in the United States include Imodium® (Ioperamide hydrochloride, piperidine opioid), Lomotil® (diphenoxylate hydrochloride with atropine sulfate, diphenoxylate being related to the narcotic meperidine), sold by G.D. Searle & Co., and Pepto-Bismol® (a bismuth subsalicylate composition) sold by Proctor and Gamble.

While opioids may be effective in treating moderate to severe diarrhea, they should not be used in patients with ulcerative colitis or acute bacillary or amebic dysentery because they appear to potentiate ulcerating processes in the colon and can provoke the development of toxic megacolon. Young children have a varying degree of blood-brain barrier maturation and are, thus, at risk of accidental overdosage, which can lead to unacceptable consequences and sometimes fatal intoxication. Additionally, the motility agents, such as Imodium®, work by the mechanism of slowing down motility in the intestines. If used too much or for too long, these agents can cause constipation. They should not be used in children under 12 years of age due to dangers of stopping normal gut function in individuals with immature immune systems.

Optimal anti-diarrheal therapies for the elderly and pediatric population are still lacking. Current management of diarrhea relies on electrolyte and fluid replacement which do not treat the underlying cause of the diarrhea.

Albumin tannate tablets have been available since the early 1900's for the treatment of diarrhea. For example, Tannalbin is a compound of tannic acid and egg-albumin. It is manufactured in Germany and was formerly sold in the United States by Merck & Co. Theoretically, Tannalbin and the other known albumin tannates, e.g. Protan, Albutannin, Gallogen, Acetannin Calco, are somewhat insoluble in gastric juice and, therefore, do not disturb the stomach. When, however, they reach the intestines, the alkaline environment dissolves the compound and the tannic acid is set free to act as an astringent on the intestinal walls and the contents of the intestines. All of the above albumin tannate compositions have been available only in solid dosage forms, such as tablets.

Children pose many challenges when it comes to taking oral medications. In general, they often resist taking medications that have an unpleasant taste or texture. In particular, young children are unable to swallow tablets or capsules. A palatable formulation is more likely to improve compliance and minimize spillage or waste during administration. Thus, there is a need for an albumin tannate suspension which is palatable, especially for children.

Acute diarrhea in adults is one of the most common diagnoses in general practice, Public Health 1994; 108: 61-8, and is responsible for considerable morbidity around the world. *The World Health Report 1996: Fighting disease, fostering development.* Geneva: WHO, 1996. In the United States, about 99 million episodes of acute diarrhea occur every year in adults. Twenty-five percent of hospitalizations in the United States were due to diarrhea and 85% of the mortality associated with diarrhea occurred in the elderly (> 65 years old). Am. J. Epidemiol. 1992; 135: 281-90.

Acute diarrhea is a complex system that may be caused by any number of diseases, both infectious and noninfectious. Acute diarrhea, if persistent, may result in clinical dehydration which may require oral rehydration therapy (ORT).

Diarrhea often develops as a side-effect disorder during the administration of cancer therapeutics. This particular diarrhea is often so serious that the patients can no longer continue to receive their cancer therapeutic treatments. These patients often have compromised digestive tracts and have difficulty taking solid medications such as tablets. Thus, there is a growing need for the development of a liquid or semi-solid dosage form for patients with compromised digestive systems.

Many of the anti-diarrheals on the market can be toxic if administration is not carefully monitored. This is especially true for children with diarrhea. Thus, there is a need for an anti-diarrheal preparation that is an all natural product which is safe for patients of all ages.

There are many situations where it would be prudent to prevent the occurrence of diarrhea. For example, some people experience traveler's diarrhea when visiting developing countries in particular due to inadequate hygiene and public sanitation. Food or beverages contaminated with bacteria, viruses, and/or parasites (such as protozoa) unfamiliar to tourists are the usual culprits, with bacteria accounting for the majority of infections. Consequently, there is a need to prevent the onset of diarrhea.

There are no recommended prophylactics for diarrhea. Antibiotics only increase a traveler's risk for adverse reactions and for infections with resistant organisms. Bismuth subsalicylate is known to decrease the incidence of diarrhea, however, its use for prophylaxis is not recommended because of potential adverse side effects and interactions with other medications.

For travelers, in addition to the usual precautions, such as avoiding tap water and raw fruits and vegetables washed with tap water, there is a need for a liquid or semi-solid composition of albumin and tannic acid that can be used as a prophylactic.

### SUMMARY OF THE INVENTION

The present invention provides a novel suspension for administration of albumin and tannic acid to maintain gastrointestinal homeostasis and minimize the occurrence of dehydration resulting from an increase in frequency, liquidity, and volume of bowel movements.

The present invention is also directed to the preparation of an albumin and tannic acid suspension for ease of administration to children or the elderly.

### DETAILED DESCRIPTION OF THE INVENTION

Namely the present invention is directed to a composition comprising albumin and tannic acid in a suspension. It will be understood the term suspension is intended to include liquids and semi-solid compositions such as gels, creams, pastes, dispersions and dilute suspensions.

One aspect of the present invention is described below in Example 1. The composition contains 125.0 mg albumin and 125.0 mg Tannic Acid /5 mL suspension. The yield is 200 liters.

### Example 1

| **COMPOSITION:** | |
|---|---|
| **Raw Material** | **% w/v** |
| Albumin | 2.500 |
| Tannic Acid | 2.500 |
| Sucrose | 10.000 |
| Sucralose | 0.200 |
| Glycerin, USP | 10.000 |
| Xanthum Gum, NF | 0.300 |
| Sodium Citrate Dihydrate, USP | 1.000 |
| Citric Acid, USP | 0.300 |
| Methylparaben, NF | 0.200 |
| Vanilla Extract #3505-001 G | 1.000 |
| Artificial Strawberry Flavor 789-780 | 2.200 |
| Purified Water, USP | Diluent |
| 6N Hydrochloric Acid | pH as needed |
| 6N Sodium Hydroxide | pH as needed |
| **Total:** | 100.00% |

The albumin is weighed to give the target weight of 5.000 kg, taking into consideration the % purity and the % moisture of the albumin raw material. The preferred albumin raw material is ovalbumin. Tannic acid is weighed to give the target weight of 5.000 kg, also taking into consideration the % purity and the % moisture of the tannic acid raw material. While many sources of tannic acid may be used, a preferred tannic acid raw material is Tanal 02C tannic acid (Tanal is a brand name owned by OmniChem, 02C is a specific grade.) The following ingredients: xanthan gum; sucrose; sucralose; citric acid; sodium citrate dihydrate; and methylparaben are weighed to achieve their target weights.

To prepare the suspending medium, 100 kg of purified water, USP, is placed into a 50 gallon tank. While mixing, the pre-weighed citric acid is added and mixed until dissolved, followed by the addition of the pre-weighed sucralose, sucrose, and sodium citrate. After the addition of each of the aforementioned ingredients, the contents of the 50 gallon tank are mixed until each added ingredient is dissolved.

In a separate 6 liter mixing tank, add 6 kg of glycerin, then add the pre-weighed xanthan gum and mix until well dispersed. The dispersed xanthan gum in glycerin is added to the 50 gallon tank. The 6 liter tank is rinsed three times with 3 liters of purified water which is added to the 50 gallon tank. 9 kg of glycerin is added to the 50 gallon tank and mixed. The pre-weighed albumin is added to the 50 gallon tank and mixed until dissolved. While continuing to mix, transfer the pre-weighed tannic acid to the 50 gallon tank and mix for 15 minutes. Add 2.000 kg of vanilla extract and 4.400 kg of artificial strawberry flavor to the 50 gallon tank and continue mixing for a minimum of 5 minutes.

In a 6 liter tank add 5 kg of glycerin and begin mixing the glycerin while slowly adding the pre-weighed methylparaben. Mix until thoroughly dispersed and transfer to the 50 gallon tank. Mix for a minimum of 30 minutes. Measure the pH and it should be in the range of 3.0 - 7.0, more preferably in the range of 4.0 - 6.0. It may be necessary to adjust the pH to the desired range with either 6N hydrochloric acid or 6N sodium hydroxide, whichever is needed. Dilute the suspension in the 50 gallon mixing tank by adding purified water to 212.0 kg, continue to mix for a minimum of 30 minutes. The suspension is now ready for dispersing into the appropriate containers for distribution to the marketplace without any further purification or isolation steps.

The finished suspension contains 125.0 mg of albumin and 125.0 mg tannic acid per 5 ml of suspension.

### Example 2

| **COMPOSITION:** | |
|---|---|
| **Raw Material** | **% w/v** |
| Albumin | 0.150 |
| Tannic Acid | 0.150 |
| Sucrose | 10.000 |
| Sucralose | 0.200 |
| Glycerin, USP | 10.000 |
| Xanthum Gum, NF | 0.300 |
| Sodium Citrate Dihydrate, USP | 1.000 |
| Citric Acid, USP | 0.300 |
| Methylparaben, NF | 0.200 |
| Banana Flavor | 2.200 |
| Purified Water, USP | Diluent |
| 6N Hydrochloric Acid | pH as needed |
| 6N Sodium Hydroxide | pH as needed |
| **Total:** | 100.00% |

The preparation of the above Example 2 suspension is similar to that described in Example 1.

The finished suspension contains 7.5 mg of albumin and 7.5 mg tannic acid.

### Example 3

| **COMPOSITION:** | |
|---|---|
| **Raw Material** | **% w/v** |
| Albumin | 45.000 |
| Tannic Acid | 45.000 |
| Sucralose | 0.200 |
| Xanthum Gum, NF | 0.300 |
| Sodium Citrate Dihydrate, USP | 1.000 |
| Citric Acid, USP | 0.300 |
| Cotton Candy Flavor | 1.000 |
| Purified Water, USP | Diluent |
| 6N Hydrochloric Acid | pH as needed |
| 6N Sodium Hydroxide | pH as needed |
| **Total:** | 100.00% |

The preparation of the above Example 3 suspension is similar to that described in Example 1.

The finished suspension has a consistency of a paste and contains 2250 mg albumin and 2250 mg tannic acid.

Another aspect of the invention is described below in Example 4.

### Example 4

| **COMPOSITION:** | |
|---|---|
| **Raw Material** | % w/v |
| Albumin | 2.500 |
| Tannic Acid | 2.500 |
| Glycerin, USP | Diluent |
| Xanthum Gum, NF | 0.300 |
| Methylparaben, NF | 0.200 |
| Grape Flavor | 2.000 |
| **Total:** | 100.00% |

The preparation of the above Example 4 is similar to that described in Example 1 with the exception that glycerin is used as the diluent instead of water, providing a gel consistency.

Only preferred embodiments of the invention and several examples of its versatility are shown and described in the present disclosure. It is to be understood that the invention is not only capable of use in various other combinations and environments, but also of changes of modifications within the scope of the inventive concept as expressed herein.

## Claims

1. A composition for maintaining gastrointestinal homeostasis in humans comprising albumin and tannic acid in a suspension.

2. The composition of claim 1, wherein the suspension comprises 0.3% - 45% albumin and 0.3% - 45% tannic acid per 5 ml.

3. The composition of claim 2, wherein the suspension comprises 2.5% albumin and 2.5% tannic acid per 5 ml.

4. A composition according to any one of claims 1-3, wherein the suspension further comprises a non-aqueous diluent.

5. The composition of claim 4 wherein the non-aqueous diluent is glycerin.

6. A composition according to any one of claims 1-5, wherein the suspension further comprises the addition of acceptable sweeteners.

7. A composition according to any one of claims 1-6, wherein the suspension further comprises the addition of flavoring agents.

8. The composition of claim 7, wherein the flavoring agents are selected from vanilla extract, bubble gum, grape, strawberry banana, orange creamsicle, cherry, grape bubble gum, cherry bubble gum, cotton candy, apple melon, chocolate, orange, artificial strawberry and banana.

9. A composition according to any one of claims 1-8, wherein the suspension has a pH range of 3.0 - 9.5.

10. A composition according to any one of claims 1-8, wherein the pH range is 4.0 - 8.0.

11. A composition according to any one of claims 1-9, wherein the pH range is 4.5 - 6.5.

12. A process for preparing a suspension of albumin and tannic acid comprising the steps of:
mixing albumin and tannic acid in a solution; and
adding selected excipients to yield a composition that can be incorporated into a dosage form without further purification or isolation.

13. The process of claim 12 further comprising the step of adding a flavoring agent.

14. A process according to claim 12 or 13, further comprising the step of adding acceptable sweeteners.

15. A process according to any one of claims 12-14, further comprising the step of maintaining the pH of the suspension in the range of 3.0 - 7.0.
